# EUROPEAN PATENT APPLICATION

(11) **EP 3 266 442 A1**
(43) Date of publication of application: **10.01.2018**
(21) Application number: 16001478.3
(22) Date of filing: 04.07.2016
(51) Int. Cl.: A61K 8/26, A61Q 19/00, A61K 8/06

(54) **EMULSION STABILIZED BY CLAY**

(71) Applicant: Clariant International Ltd, 4132 Muttenz (CH)
(72) Inventor: SOHLING, Ulrich, 85356 Freising (DE)
(74) Representative: Hütter, Klaus

(57) **Abstract**

The invention relates a composition in form of a stable oil-in-water emulsion comprising
a) at least one natural clay which belongs to the class of saponites clay minerals and has a cation exchange capability equal or lower than 40 meq per 100 g of dry clay,
b) a dispersed oil component, and
c) an aqueous continuous phase.

## Description

This invention relates to an oil-in-water emulsion stabilized only by smectite clay mineral which has excellent emulsion stability in a broad range of pH and salt concentration.

Emulsions are colloidal systems where droplets of one liquid are dispersed in a second non-miscible liquid. Though they are thermodynamically unstable and tend to separate, they can be stabilized by surfactants, amphiphilic molecules which adsorb at the liquid interface and reduce the interfacial tension between the immiscible liquids. Alternatively, solid particles can also be used to stabilize emulsions, provided that they fulfill the partial wetting conditions, that means, the particles are wetted by both liquids and therefore adsorb at the interface, leading to a reduction of the liquid interfacial area of the system. Specific benefit derived from the stabilization of emulsions by particles is the high resistance against coalescence due to the practically irreversible attachment of the particles at the interface. This feature becomes of special interest in application fields like enhanced oil recovery or agrochemical formulations, where high temperature and/or salinity dominates and surfactants usually show a lack of performance, as described in WO-2014/114538 and WO-2015/170099. Moreover, the formulation of "surfactant-free" emulsions allows to overcome adverse effects related to the use of surfactants in certain applications, like skin irritancy in topical formulations, as described in International Journal of Pharmaceutics 428 (2012) 1 - 7.

Clay minerals have a sheet-like structure and are composed of tetrahedral layers of silicate linked to octahedral layers of metal oxides. Depending on the number of layers linked together building a structural motif, clays are classified in 1:1 phyllosilicates, which consist of one tetrahedral layer of silica bond through oxygen atoms to one octahedral layer of metal (aluminium or magnesium) oxide, 2:1 phyllosilicates, which consist of octahedral metal (aluminium or magnesium) oxide layer sandwiched between 2 tetrahedral layers of silica, and 2:1 inverted ribbons, which present continuous fibrous-like tetrahedral sheets together with discontinuous octahedral sheets.

Smectites are a group of 2:1 phyllosilicates characterized by the partial substitution of cations in the layers by other cations of lower charge, thus originating an excess of negative charge in the layers which is compensated by cations intercalated between the sheets. Typically the degree of substitution and therefore of charge is low for the members of the smectite family. A further differentiation of smectites is done regarding either the oxygen atoms in the octahedral oxide layer are coordinated bridging two metal centers (dioctahedral smectites, the metal is typically Al³⁺) or each oxygen atom is shared by three metal centers (trioctahedral smectites, the metal is typically Mg²⁺).

Montmorillonite (Z_{X}[Al₂³⁺]^{oct}[Si₄]t^{etr}O₁₀(OH)₂) is the main representative class of the dioctahedral smectites. It consists of an octahedral layer of aluminum oxide sandwiched between two silica tetrahedral lays platelet-like monocrystals with a constant thickness of 1.1 nm and a diameter between 50 - 1000 nm. The platelets are typically stacked in large piles. The substitution of aluminium atoms by magnesium atoms in the octahedral layer or silicon atoms by aluminum atoms in the tetrahedral layer creates a net negative charge in the crystals, which is balanced by the presence of counter ions (typically sodium or calcium) in the interlayer gallery. (Z_{X}[Mg₃²⁺]^{oct}[Si₄]t^{etr}O₁₀(OH)₂) is the basic structure of trioctahedral smectites. When the magnesium ions are partially substituted by lithium ion, the clay is called laponite (or hectorite), while when the tetrahedral silicon are partially replaced by aluminium, the clay is called saponite.

The cation exchange capacity of a clay (CEC) is a well-known parameter used to define the concentration of negatively charged sites on the clay sheets that can adsorb exchangeable cations. The higher the CEC value, the higher the anionic charge on the clay crystals. Typically, the CEC can be determined using the ammonium chloride method, as described for example in Clay Minerals, 44 (2009), 525 - 537 and is indicated in terms of miliequivalents per 100 g of dry clay.

FR-2976503 discloses oil-in-water emulsions stabilized by clay minerals previously treated with organic compounds in order to render the particles more hydrophobic and thus increase their affinity towards the oil phase. The pre-treatment of the clays with the organic compounds is an additional processing step. For environmental and economic reasons it would be of interest to find a clay which can stabilize oil-in-water emulsions without need of a hydrophobizing pre-treatment with organic compounds.

The stabilization of mixtures of monolinolein and different kind of oils (glycerol trioleate, R-(+)-limonene and styrene) by two unmodified smectites (purified Na⁺-montmorillonite and synthetic laponite) is described in Journal of Colloid and Interface Sciences, 333 (2009) 563-569. The clays are claimed to act as cosurfactants in the sense that they support monolinolein, which is an amphiphilic molecule and act as emulsifier, to stabilize the oil-in-water emulsions. Either the emulsions can be stabilized in the absence of surfactant solely by the clays or the stability of the described emulsions is perturbed by the addition of electrolyte to the aqueous phase is not disclosed.

Stable emulsions of paraffin oil in water stabilized by combinations of smectites and non-ionic surfactants are disclosed in Applied Clay Science, 14 (1999) 83 - 103. Especially Na⁺ montmorillonite with a cation exchange capacity of 103 meq per 100 g dry clay is mentioned to form stable emulsions. The addition of sodium chloride or calcium chloride to the aqueous phase is reported to stabilize the emulsions against coalescence. No information about the ability of the clays to stabilize the emulsions in absence of surfactants is disclosed.

Nevertheless there is an on-going need to provide solid materials able to stabilize oil-in-water emulsions in the absence of amphiphilic molecules. Such materials have to be economically competitive and environmentally sustainable, that means they must be easy to be produced and do not be classified as hazardous chemicals.

Unexpectedly it was found that natural clays which belong to the class of saponites or are natural blends of saponite and kerolite and have a cation exchange capacity equal or lower than 40 meq per 100 g of dry clay are able to stabilize oil-in-water emulsions without addition of any surfactant.

The clays disclosed in this invention are natural raw materials which due to their unexpected unique surface properties can efficiently adsorb at the oil-water interface and prevent oil droplets from coalescence without the need of a hydrophobizing pre-treatment with organophilic molecules.

Surprisingly, the clays being object of this invention stabilize oil-in-water emulsions without additional stabilizers such as surfactants being needed. Oil-droplet stabilization in the aqueous phase and prevention of coalescence and phase separation is fulfilled by the clays disclosed in this invention.

Furthermore it was also unexpectedly found that the clays disclosed in this invention are able to stabilize oil-in-water emulsions against coalescence at diverse pH values and salt concentrations.

The oil-in-water emulsion is considered to be stable if coalescence and phase separation does not occur for at least three months after preparation during storage at temperatures around room temperature (23 °C). Coalescence can be tracked by changes in the droplet size during time and phase separation can be visually evaluated.

The present invention is related to oil-in-water emulsions stabilized solely by natural clays which belong to the class of saponites or are natural blends of saponite and kerolite and have a cation exchange capability equal or lower than 40 meq per 100 g of dry clay.

The instant invention therefore relates to a composition in form of a stable oil-in-water emulsion comprising
a) at least one natural clay which belongs to the class of saponites clay minerals and have a cation exchange capability equal or lower than 40 meq per 100 g of dry clay,
b) a dispersed oil component, and
c) an aqueous continuous phase

Natural clays are extracted from geological deposites. Natural clays occur typically as a mixture of diverse components, such as clay minerals, metal oxides and other weathered minerals. Preferred natural clays according to the present invention belong to the class of the saponites. Most preferred clays according to the present invention occur as a natural blend of saponite and kerolite.

Preferred clays (component a) suitable to formulate compositions in accordance with the present invention have a cation exchange capacity from 1 to 35 meq per 100 g of dry clay. Particularly preferred clays have a cation exchange capacity from 15 to 35 meq per 100 g of dry clay.

The clays have preferably a BET-surface area from 90 to 300 m²/g. Particularly preferred clays have a BET-surface area from 105 to 275 m²/g. Most preferred clays have a BET-surface area from 120 to 250 m²/g.

Preferred clays have a surface free energy from 10 to 50 mN/m. Particularly preferred clays have a surface free energy from 20 to 45 mN/m.

The concentration of clay in the composition in accordance with the present invention is preferably from 0.01 % to 15 % by weight, more preferably from 0.1 % to 10 % by weight and most preferably from 0.3 % to 5 % by weight.

The composition of the present invention contain at least one oil component (component b), which is any fatty substance which is liquid at room temperature (25 °C). Among the oils that can be used in the emulsion of the invention, mention may be made, for example, of plant oils such as sunflower, olive, soybean, rapeseed, canola, jojoba, palm, peanut, avocado, soft-almond, apricot and corn oils and the liquid fraction of karite butter; chemically modified plant oils such as methylated or ethylated seed oils and medium-chain triglycerides; hydrocarbon-based oils chosen from linear or branched hydrocarbons of mineral or synthetic origin, preferably from a volatile or nonvolatile liquid paraffin oil, hydrogenated isoparaffin, naphthalene oil, a totally or partially hydrogenated liquid polydecene, isoeicosane, a decene/butene copolymer, or a polybutene/polyisobutene copolymer, and mixtures thereof. Synthetic oils such as 2-ethylhexyl palmitate, isopropyl myristate, isononyl isononanoate and cetearyl octanoate; volatile or non-volatile silicone oils and fluorinated oils.

The concentration of oil component in the composition in accordance with the present invention is preferably from 0.5 % to 60 % by weight, more preferably from 1 % to 45 % by weight and most preferably from 5 % to 30 % by weight.

For the skilled person in the art results obvious that liphophilic active ingredients can be dissolved in the oil phase and be thus formulated in form of a stable oil-in-water emulsion in accordance with the presence invention. Among the active ingredients that can be formulated, mention may be made, for example, of cosmetic ingredients such as essential oils, UV-filters and anti-aging agents, and agrochemical agents such as pesticides.

In the aqueous phase (component c) of the emulsion different additives can be present, such as rheology modifiers, pH control agents, chelating agents, electrolytes, biocides and humectants.

The oil-in-water emulsions solely stabilized by clays according to this invention are stable in a broad range of pH and electrolyte concentration. Stable means that there are almost no changes in the droplet size of the dispersed oil phase when pH and/or salt concentration in the continuous aqueous phase of the emulsion is varied, and no phase separation is observed.

In the compositions of the present invention, typically there is no need for the presence of conventional emulsifiers in the form of amphiphilic molecules in order to achieve stabilization of the dispersed oil phase. If used, the amphiphilic molecules are present in an amount of at most 0.5 % by weight and most preferably at most at the critical micellar concentration, and they are incorporated in the formulation to optimize the wettability of the emulsion by lowering of the dynamic surface tension of the system.

Another embodiment of the present invention is also related to a process for the production of an oil-in-water emulsion with a composition according to the described above. The emulsion can be prepared by using any of the emulsification techniques described elsewhere, such as for example, high pressure homogenization, rotor-stator mixing, cavitation, membrane emulsification and microfluidic techniques. In a preferred manufacturing process, the clay (component a) is dispersed in water under high mechanical shear generated by a rotor-stator mixer. The oil phase is added to the aqueous clay dispersion and the composition is homogenized with a rotor-stator mixer in order to obtain a stable oil-in-water emulsion.

The preferred droplet size of the emulsions can range from 1 µm to 200 µm. Specially preferred droplet size of the emulsion is in the range from 10 µm to 150 µm. Most preferred droplet size of the emulsion is in the range from 15 to 100 µm. This droplet size refers to the discontinuous phase of the emulsion and is determined by analysis of microscopic image of the emulsion.

Other embodiment of the present invention is related to the use of a composition according to the described above in personal care and cosmetics formulations, such as for example, hand creams, lotions, hair shampoos and conditioners and sunscreens; in agrochemical formulations, in emulsion paints, in cleaning formulations and in the oil & mining industry.

### Examples

### Characterization of the clays

The cationic exchange capacity (CEC) was determined following the NH₄Cl method. The clay material was dried at 150 °C for 2 h and the resulting material allowed to react under reflux with a large excess of aqueous NH₄Cl solution for 1 h. After standing at room temperature for 16 h, the material was filtered. The filter cake was washed, dried and ground, and the NH₄ content in the clay material was determined through measurement of the N content by elemental analysis using a CHN-Analyser Vario EL III by Elementar, Hanau (D).

The specific surface area was determined by the BET method (single-point method using nitrogen, according to DIN 66131) with an automatic nitrogen porosimeter by Micrometrics, type ASAP 2010.

The surface free energy was calculated applying the Owens-Wendt-Rabel-Kaelbe model using contact angles for water and diiodomethane obtained from powder contact angle measurements on the clays with a Krüss Force Tensiometer K100 following the Washburn method. For clays swelling rapidly in contact with water, ethanol was used instead of water for the contact angle measurements.

| Clay | CEC (meq in 100 g) | BET-surface area (m²/g) | Surface free energy (mN/m) | Mineral class |
|---|---|---|---|---|
| A | 25 | 125 | 24 | natural saponite |
| B | 20 | 224 | 44 | natural blend of saponite with kerolite |
| C (comparison) | 52 | 239 | 55 | natural blend of montmorillonite with amorphous silica |
| D (comparison) | 94 | 83 | 48 | natural montmorillonite |
| E (comparison) | 49 | 67 | | natural montmorillonite |

### General procedure for preparation and characterization of emulsions

Clay particles are firstly dispersed in buffer solution by stirring at 800 rpm for 5 min with high shear mixer (Ultra Turrax T25-IKA^{®} equipped with dispersing tool consisting of S25N shaft and 25G generator) and then the oil phase is added to the dispersion and homogenized at 6500 rpm for 5 min. The total mass of the emulsion is typically 100 g.

The emulsion type was determined by the drop test. A drop of the formed emulsion was added to water. Immediate dispersion of the droplets confirmed the presence of oil-in-water emulsion type.

The stability of the emulsion was evaluated from the visual inspection and assessed according to the volume of emulsion formed and the speed of phase separation after preparation and after 90 days at room temperature. According to the volume of each phase (especially the oil separation), the stability of the emulsion was assessed. The more oil release pointed to the instability of the emulsion.

The morphology and size of the emulsion droplets was observed by optical microscope (Olympus DP26, Japan) and the droplet size determined by analysis of microscopic image of the emulsion.

For comparison of the emulsification efficiency, clays A, B, C, D and E were used at a concentration of 1 wt.-% in citrate buffer pH 5.5 to prepare emulsions of Myritol 318 (medium chain triglyceride, BASF) at a volume fraction of 20 %. The volumen of the existing phases (separated oil phase, (creamed) emulsion, aqueous phase and solid sediment) was recorded after preparation (D1) and after 90 days storage at room temperature (D90)

| | A | | B | | C | | D | | E | |
|---|---|---|---|---|---|---|---|---|---|---|
| | D1 | D90 | D1 | D90 | D1 | D90 | D1 | D90 | D1 | D90 |
| oil phase (vol.-%) | 0 | 0 | 0 | 0 | 7 | 13 | 48 | 48 | 2 | 4 |
| emulsion (Vol.-%) | 81 | 56 | 49 | 40 | 34 | 31 | 0 | 0 | 37 | 33 |
| aqueous phase (vol.-%) | 18 | 44 | 40 | 51 | 52 | 49 | 52 | 12 | 61 | 48 |
| sediment (vol.-%) | 1 | 1 | 11 | 9 | 7 | 7 | 0 | 40 | 0 | 15 |

Clays A and B form very stable emulsions and no separation of oil was observed after 90 days at room temperature. Compression of the emulsion volumen due to creaming occured over the storage time which was due to the density differences between the bulk and the dispersed phases, but the oil droplets remained dispersed indicating that the emulsion was stable.

For comparison, clay C and E showed oil separation after preparation, indicating that these clays were not as efficient for emulsion stabilization as A and B. Moreover, the volume of separated oil phase increased during storage. Clay D was not able at all to stabilize oil droplets and phase separation occurred after preparation.

To check the influence of salt on the stability of the emulsions, samples were prepared using the same conditions as mentioned before but adding increasing concentrations of sodium chloride. The droplet size of the resulting emulsions was compared.

| NaCl (wt.-%) | Average droplet size (µm) | | |
|---|---|---|---|
| | A-1753 | B-1694 | E-0075 |
| 0 | 25 | 45 | 107 |
| 0.5 | 25 | 46 | 170 |
| 1.0 | 24 | 47 | 342 |

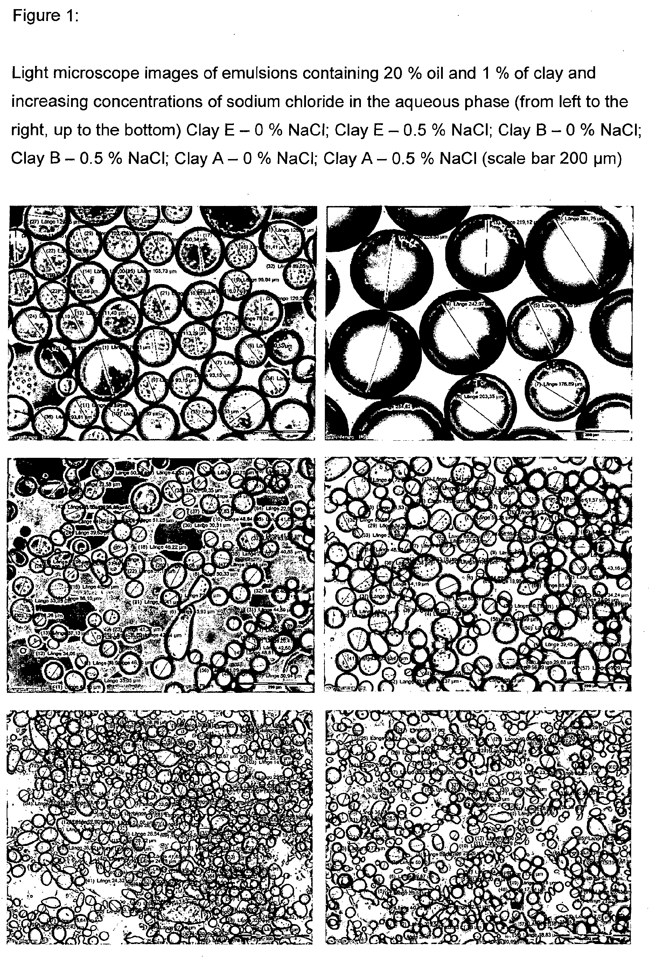

The droplet size of emulsions prepared with clays A and B remained almost invariable independently of the salt concentration in the aqueous phase, whereas the droplet size increased notably with the salt concentration for clay E (see figure 1).

The same kind of experiment was repeated but changing the pH value, which was adjusted to 4 and 9 using respective citrate buffers. Again clays A and B were able to stabilize the emulsions at both acidic and alkaline pH, whereas clay E was not able to form any emulsion at pH 9 and phase separation was immediately found after preparation.

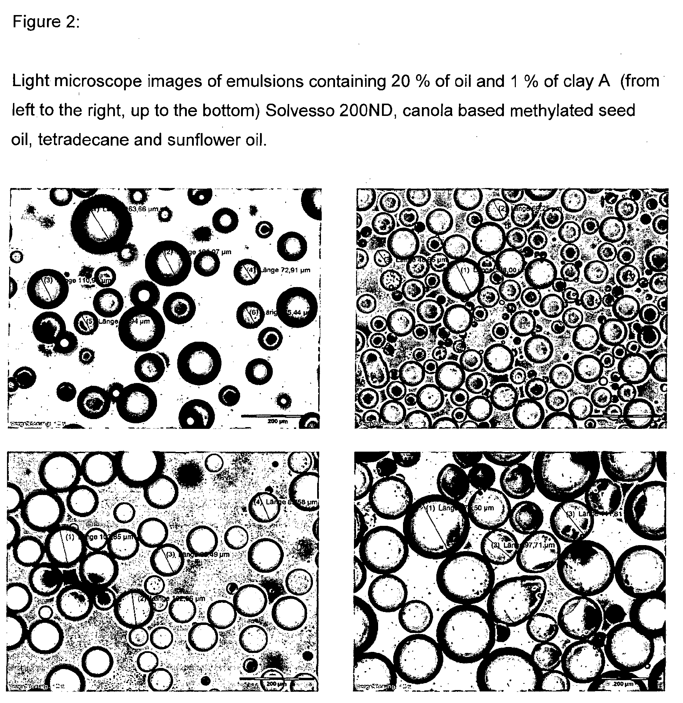

In order to show the ability of the clays according to this invention to stabilize emulsions of diverse types of oils, quick emulsification tests were done by dispersing clay A in citric buffer pH 5.5 by vortex mixing (IKA 4 Digit) at 3000 rpm for 20 s, adding the oil and further mixing for 40 s at the same speed. Clay concentration was 1 wt.-% and the oil volumen fraction 20 %. Oils used were Solvesso 200ND (mixture of aromatic hydrocarbons, Exxonmobil), Canola based methylated seed oil (Oleon), tetradecane (Alfa Aesar) and sunflower oil. Stable emulsions were obtained for all type of oils (see figure 2).

Furthermore, in order to show that the ability of stabilizing oil-in-water emulsions by the clays according to this invention is not affected by other typical components of a formulation, such as for example rheology modifiers, biocides and humectants, an oil-in-water emulsion containing 1 wt.-% of clay A, 20 wt.-% of medium chain triglyceride (Myritol 318, BASF), 0.5 wt.-% of a copolymer of vinylpyrrolidone and 2-acrylamido-2-methylpropane sulfonic acid (Aristoflex AVC, Clariant), 1 wt.-% of phenoxyethanol (Phenonip ME, Clariant) and 2.5 wt.-% glycol (Merck) was prepared as follows:
Glycerol was dissolved in desionized water and clay A was added and stirred at 800 rpm for 5 min with high shear mixer (Ultra Turrax T25-IKA^{®} equipped with dispersing tool consisting of S25N shaft and 25G generator). Then Myritol 318 was added to the dispersion and homogenized at 6500 rpm for 5 min. Aristoflex AVC was then added and the mixture was homogenized for another 2 min at 9500 rpm. Finally, Phenonip ME was added to the emulsion and gently stirred for 1 min.

The obtained emulsion had the texture of typical cosmetic hand cream. The stability of the emulsion was investigated by comparison of the rheological properties of the sample after preparation and after 90 days storage at 50 °C.

All the measurements were done with a rheometer MARS III (Thermofisher Scientific) with a cone (2°, 35 mm) / plate geometry.

The flow of the emulsion was evaluated by a shear controlled sweep between 0 and 1000 1/s at 23 °C. Frequency sweeps were carried out at 23 °C to characterize the viscoelastic properties of the emulsion. After pre-shearing steps at constant shear rate of 100 1/s for 2 min followed by 0.001 1/s for 30 min, a 0.01592 - 15.92 Hz Hz ramp at constant strain of 1 % (which was preciously determined to be in the linear viscoelastic range via strain controlled amplitude sweep) was measured.

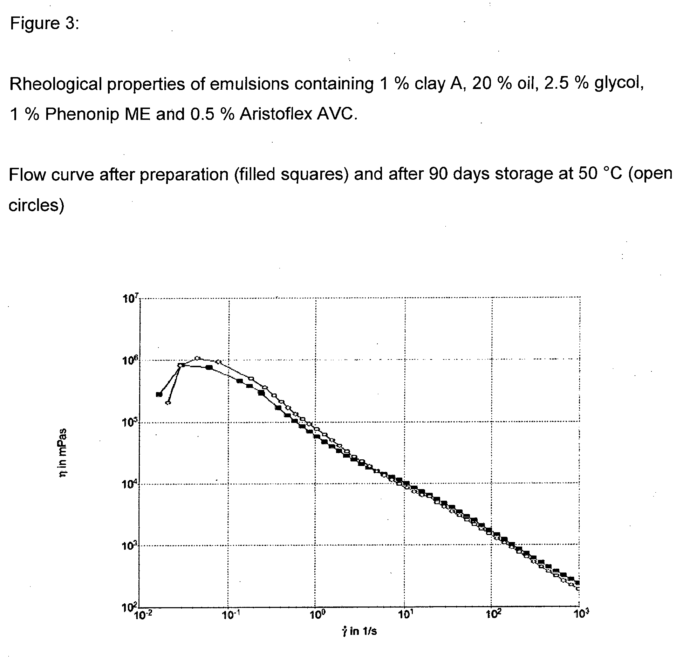

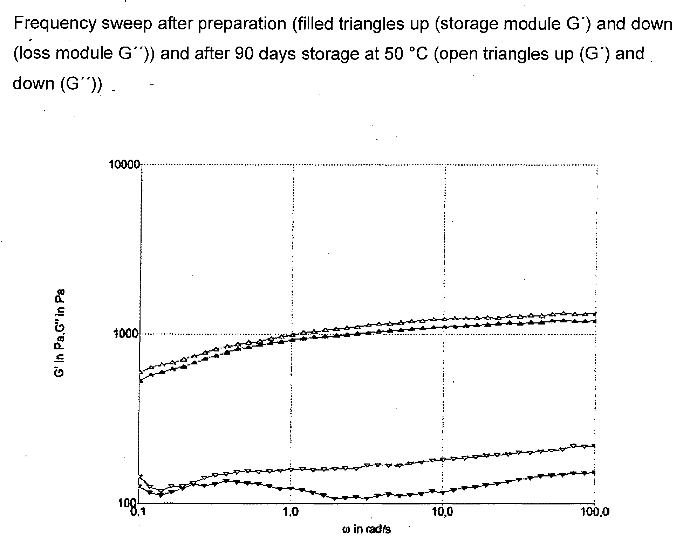

The measurements showed that the rheological properties of the emulsion remained almost constant during the storage, indicating that the system was highly stable and no changes occurred in the colloidal structure of the emulsion (see figure 3).

## Claims

1. Composition in form of a stable oil-in-water emulsion comprising
a) at least one natural clay which belongs to the class of saponites clay minerals and has a cation exchange capability equal or lower than 40 meq per 100 g of dry clay,
b) a dispersed oil component, and
c) an aqueous continuous phase.

2. Composition according to claim 1, wherein the natural clay is a natural blend of saponite and kerolite.

3. Composition according to claim 1 and/or 2, wherein the cation exchange capacity is from 1 to 35 meq per 100 g of dry clay.

4. Composition according to one or more of the preceding claims, wherein the clay has a BET-surface area from 90 to 300 m²/g.

5. Composition according to one or more of the preceding claims, wherein the clay has a surface free energy from 10 to 50 mN/m.

6. Composition according to one or more of the preceding claims, wherein the concentration of clay in the composition is in the range from 0.01 % to 15 % by weight.

7. Composition according to one or more of the preceding claims, wherein the oil component (b) is a fatty substance which is liquid at room temperature (25 °C).

8. Composition according to claim 7, wherein the oil component is made of plant oils, in particular sunflower, olive, soybean, rapeseed, canola, jojoba, palm, peanut, avocado, soft-almond, apricot and corn oils and the liquid fraction of karite butter or chemically modified plant oils, hydrocarbon-based oils or synthetic oils or volatile or non-volatile silicone oils and fluorinated oils.

9. Composition according to one or more of the preceding claims, wherein the concentration of oil component in the composition is in the range from 0.5 % to 60 % by weight.

10. Composition according to one or more of the preceding claims, wherein liphophilic active ingredients are dissolved in the oil phase.

11. Composition according to one or more of the preceding claims, wherein the aqueous phase (c) comprises different additives, in particular rheology modifiers, pH control agents, chelating agents, electrolytes, biocides and/or humectants.

12. Composition according to one or more of the preceding claims, wherein the droplet size of the emulsions is in the range from 1 µm to 200 µm.

13. Use of a composition according to one or more claims 1 to 12 for personal care and cosmetics formulations, in agrochemical formulations, in emulsion paints, in cleaning formulations and in the oil & mining industry.
